# EUROPÄISCHE PATENTSCHRIFT

(11) **EP 2 637 839 B1**
(45) Veröffentlichungstag und Bekanntmachung des Hinweises auf die Patenterteilung: **13.04.2016**
(21) Anmeldenummer: 11774000.1
(22) Anmeldetag: 14.10.2011
(51) Int. Cl.: B29C 65/02, B29C 65/74, B65B 3/28, G01G 13/24, G01G 17/06, B65B 3/00

(54) **VERWENDUNG EINER SCHWEISSVORRICHTUNG ALS STEUERVENTIL EINER DOSIERVORRICHTUNG UND DOSIERVORRICHTUNG**
USE OF A WELDING DEVICE AS A CONTROL VALVE OF A METERING DEVICE AND METERING DEVICE
UTILISATION D'UN DISPOSITIF DE SOUDURE COMME VANNE DE COMMANDE D'UN DISPOSITIF DOSEUR ET DISPOSITIF DOSEUR

(30) Priorität: 08.11.2010 DE 102010060401
(43) Veröffentlichungstag der Anmeldung: 18.09.2013
(73) Patentinhaber: Sartorius Lab Instruments GmbH & Co. KG, 37075 Göttingen (DE)
(72) Erfinder: MÜLLER, Michael, 37079 Göttingen (DE); WEITEMEIER, Swen, 37139 Lödingsen (DE); STRIETZEL, Steffen, 37308 Siemerode (DE)
(74) Vertreter: Schneider, Peter Christian
(86) Internationale Anmeldenummer: PCT/EP2011/005155
(87) Internationale Veröffentlichungsnummer: WO 2012/062395

(56) Entgegenhaltungen:
- EP-A2- 0 639 384
- WO-A1-00/62891
- DE-U1-202007 014 392
- JP-A- 9 019 495
- US-A- 3 788 374
- US-A- 5 881 535

## Beschreibung

### Gebiet der Erfindung

Die Erfindung betrifft eine Verwendung einer Schweißvorrichtung zum verschließenden Verschweißen von thermoplastischen Kunststoffschläuchen.

Die Erfindung betrifft weiter eine automatisierte Dosiervorrichtung zum dosierten Überführen eines Mediums von einem Vorratsbehälter durch einen aus thermoplastischem Kunststoff bestehenden Verbindungsschlauch in einen Zielbehälter.

### Stand der Technik

In der modernen Medizin- und Biotechnik existiert ein starker Trend weg von wiederverwendbaren Behältern hin zu Einmal-Behältern, sogenannten "Disposables". In der Regel haben alle Herstellungs-, Abfüllung-, Lagerungs- und Anwendungsprozesse medizinischer und/oder biotechnischer Flüssigkeiten unter streng sterilen Bedingungen zu erfolgen. Dies erfordert im Fall der Benutzung wiederverwendbarer Behältnisse deren Sterilisierung sowie Überprüfung und Dokumentation der erzielten Sterilität. Die damit verbundenen Prozesse sind technisch aufwendig und kostenintensiv. Sie können bei Verwendung von Einmal-Behälter, die bereits vom Hersteller steril geliefert werden, weitestgehend entfallen. Am Markt haben sich insbesondere Kunststoffbeutel als Einmal-Behälter durchgesetzt. Diese sind kostengünstig herstellbar, einfach zu sterilisieren, leicht und im leeren Zustand wenig voluminös, sodass sie einfach entsorgt werden können.

Bei typischen Prozessen sowohl bei der Herstellung als auch der Anwendung medizinischer und/oder biotechnischer Flüssigkeiten sind Dosierprozesse von besonderer Bedeutung. Sie treten bei der Applikation der Flüssigkeit ebenso auf wie beim Mischen verschiedener Flüssigkeiten oder beim Abfüllen einer Flüssigkeit in handelsübliche Gebinde. In jedem Fall muss mindestens eine Flüssigkeit aus mindestens einem Vorratsbehälter in mindestens einen Zielbehälter umgefüllt werden. Zur Verbindung der jeweils als Kunststoffbeutel ausgeführten Vorrats- und Zielbehälter haben sich flexible Kunststoffschläuche etabliert. Unter hygienischen Aspekten ist die Kopplung eines Schlauches mit einem Behälter nicht unkritisch, weshalb die Hersteller in der Regel die Beutel bereits einteilig oder stoffschlüssig mit Schläuchen oder ganzen Schlauchsystemen versehen. Diese Schläuche werden nach Abfüllung des Beutels typischerweise an ihren Enden durch Verschweißung dauerhaft geschlossen. Das verschweißende Verschließen der Schläuche hat mehrere Vorteile. Zum einen ist ein mittels Verschweißung verschlossener Beutel bzw. Schlauch manipulationssicher. Zum anderen wird das Schlauchende durch den Schweißvorgang erhitzt, sodass an der kritischen Öffnungsstelle ein zusätzlicher Sterilisationsvorgang durchgeführt wird. Die Verschweißung ist grundsätzlich bei allen thermoplastischen Kunststoffen, wie z.B. PVC, PE, PET, PU etc. möglich.

Aus der EP 0 551 813 B1 ist eine Schweißvorrichtung bekannt, bei der der zu verschweißende Schlauch zwischen zwei als Schweißelektroden ausgebildeten Klemmbacken eingeklemmt wird. Der während des gesamten Prozesses konstante Klemmdruck ist so hoch, dass er das freie Lumen des Schlauches an der Klemmstelle vollständig verschließt. Die Klemmbacken bilden gemeinsam einen Kondensator eines elektrischen Hochfrequenz-Schwingkreises. Durch Ansteuerung des Schwingkreises mit einer geeigneten Hochfrequenz werden die Moleküldipole des zwischen den Klemmbacken eingeklemmten Schlauchmaterials in Schwingung versetzt und so die Schlauchwände erhitzt. Die Erhitzung führt zum Aufschmelzen des Schlauchmaterials, sodass die Klemmbacken unter dem konstanten Klemmdruck das aufgeweichte Schlauchmaterial aus der Klemmstelle herausquetschen, sodass es zur Verschweißung und schließlich zur Durchtrennung der Schweißstelle kommt. Die genannte Druckschrift beschäftigt sich insbesondere mit der Nachregelung der Resonanzfrequenz des elektrischen Schwingkreises, die sich durch die passive Annährung der Klemmbacken unter dem konstanten Klemmdruck aufgrund der resultierenden Kapazitätsänderung des Kondensators verändert.

Eine weitere auf Hochfrequenzbasis arbeitende Schweißvorrichtung ist beispielsweise aus der JP 9 019495 A bekannt.

Aus der EP 1 525 138 B1 ist eine Dosiervorrichtung bekannt, mittels deren eine Vielzahl von kleinen Beuteln als Zielbehälter aus einem großen Beutel als Vorratsbehälter abgefüllt werden können. Die Dosierung, d.h. die Steuerung des Volumenstroms aus dem Vorratsbehälter zu den Zielbehältern erfolgt mittels Klemmventilen, die die Schlauchverbindungen zwischen dem Vorratsbehälter und den Zielbehältern zwischen aktiv steuerbaren Klemmbacken einklemmen. Dabei wird der Klemmdruck gesteuert variiert, sodass sich das freie Lumen des jeweiligen Schlauches an der Klemmstelle verändert und der Volumenstrom durch den Schlauch an der Klemmstelle entsprechend variiert werden kann. Nach Beendigung des Abfüllvorgangs wird der Zugangsschlauch des jeweiligen Zielbeutels mit einer Verschlussklemme verschlossen und kann zusätzlich, beispielsweise mit der aus der EP 0 551 813 B1 bekannten Schweißvorrichtung, dauerhaft verschweißt werden.

Weitere Dosiervorrichtungen, bei denen die zwischen dem jeweiligen Zielbehälter und einem Vorratsbehälter angeordneten Verbindungsschläuche nach dem Befüllvorgang der Zielbehälter mittels einer externen Schweißvorrichtung dauerhaft verschweißt werden, sind beispielsweise aus der WO 00/62891 A1 und der DE 20 2007 014 392 U1 bekannt.

Es sind außerdem Dosiervorrichtungen bekannt, bei denen eine Schweißvorrichtung für die Verschweißung der Verbindungsschläuche bereits in der Dosiervorrichtung selbst integriert ist. Eine solche Dosiervorrichtung wird beispielsweise in der EP 0 639 384 A2 beschrieben.

Bei der aus der EP 0 639 384 A2 bekannten Dosiervorrichtung erfolgt der Flüssigkeitstransport mittels einer Rollenpumpe oder durch das Zusammendrücken des Vorratsbehälters, wodurch die darin enthaltene Flüssigkeit über einen Verbindungsschlauch in einen Zielbehälter überführt wird. Dabei kann der Volumenstrom am Verbindungsschlauch durch einen Klemmdruck zweier Klemmbacken gesteuert werden. Nach Beenden des Dosiervorgangs werden zwei zusätzliche, separate, bewegbare Klemmbacken durch eine Heizeinrichtung auf eine konstante Temperatur aufgeheizt und währenddessen so aufeinander zubewegt, dass sie den Schlauch eindrücken und verschweißen. Nach einer bestimmten Zeit werden beide Klemmbacken voneinander entfernt. Die verschweißenden Klemmbacken werden dabei durch einen Aktuator gesteuert, der mit einer Steuereinheit gekoppelt ist. Eine separate Trennvorrichtung trennt den Verbindungsschlauch an der Schweißstelle.

Eine weitere Dosiervorrichtung mit integrierter Schweißvorrichtung ist aus der US 5 881 535 A bekannt. Bei dieser Dosiervorrichtung wird zunächst in einer Dosierstation eine Flüssigkeit von einem Vorratsbehälter durch einen Verbindungsschlauch in einen Zielbehälter mit Einlassschlauch überführt und anschließend der Einlassschlauch in einer Schweißstation durch Verschweißen verschlossen. Der Transport der Flüssigkeit wird dabei durch eine Spritzenkolbenpumpe bewerkstelligt. Der Verbindungschlauch kann durch einen pneumatischen Aktuator gegen ein Gegenstück gedrückt werden, um den Verbindungsschlauch nach Art eines binären Klemmventils vollständig zu verschließen oder zu öffnen. Nach dem Dosiervorgang wird der Einlassschlauch des Zielbehältnisses verschweißt. Eine bewegbare Klemmbacke der gesonderten Schweißstation wird dazu so auf eine statische Klemmbacke zugeführt, dass der Einlassschlauch, der sich zwischen beiden Klemmbacken befindet, und ein Blasebalg zusammengedrückt werden. Bei einem vordefinierten Innendruck des Blasebalgs startet eine Heizeinrichtung, die den Schlauch durch Vibration der bewegbaren Klemmbacke erhitzt und mittig verschweißt. Nach einer definierten Zeit stoppt der Vibrationsmechanismus und der Druck zwischen bewegbarer Klemmbacke und nicht bewegbarer Klemmbacke wird gehalten, sodass das Zielbehältnis während des Abkühlvorgangs hermetisch verschlossen bleibt.

Nachteilig bei den bekannten Dosiervorrichtungen ist, dass die Verschweißung der Zugangsschläuche für die Zielbehälter in einem separaten Arbeitsschritt erfolgen muss, was neben den erhöhten Kosten durch Zeitaufwand und die Notwendigkeit einer zusätzlichen Schweißvorrichtung stets auch zusätzliche hygienische Risiken birgt.

### Aufgabenstellung

Es ist die Aufgabe der vorliegenden Erfindung, eine kosteneinsparende und die hygienische Sicherheit verbessernde Dosiervorrichtung zur Verfügung zu stellen.

### Darlegung der Erfindung

Die oben genannte Aufgabe wird gemäß Anspruch 1 gelöst, durch die Verwendung einer Schweißvorrichtung zum verschließenden Verschweißen von thermoplastischen Kunststoffschläuchen, umfassend eine Klemmvorrichtung mit wenigstens zwei Klemmbacken, von denen wenigstens eine bewegbar ist und zwischen denen ein zu verschweißender Schlauch einklemmbar ist,
wobei die Klemmvorrichtung eine mit einer Steuereinheit gekoppelte Heizeinrichtung zum Erhitzen des zwischen den Klemmbacken eingeklemmten Schlauches aufweist, und
die wenigstens eine bewegbare Klemmbacke mittels eines steuerbaren, bidirektionalen Aktuators betätigbar ist, der derart mit der Steuereinheit gekoppelt ist, dass der auf den Schlauch wirkende Klemmdruck unabhängig von der Heizeinrichtung einstellbar ist,
als ein steuerbares Klemmventil einer automatisierten Dosiervorrichtung, mittels derer ein Medium von einem Vorratsbehälter durch einen zwischen den Klemmbacken eingeklemmten, aus thermoplastischem Kunststoff bestehenden Verbindungsschlauch dosiert in einen Zielbehälter überführbar ist.

Hieraus ergibt sich eine automatisierte Dosiervorrichtung gemäß Anspruch 4 zum dosierten Überführen eines Mediums von einem Vorratsbehälter durch einen aus thermoplastischem Kunststoff bestehenden Verbindungsschlauch in einen Zielbehälter ,
wobei der Verbindungsschlauch zwischen wenigstens zwei Klemmbacken, von denen wenigstens eine bewegbar ist, einer mit einer Steuereinheit gekoppelten, steuerbaren Klemmvorrichtung einer Schweißvorrichtung, positionierbar ist, sodass ein Volumenstrom des Mediums durch Steuerung eines von der Klemmvorrichtung an einer Klemmstelle auf den Verbindungsschlauch ausgeübten Klemmdrucks steuerbar ist, wobei wenigstens eine der Klemmbacken eine mit der Steuereinheit gekoppelte Heizeinrichtung zum Erhitzen des Verbindungsschlauches aufweist und die wenigstens eine bewegbare Klemmbacke mittels eines steuerbaren, bidirektionalen Aktuators betätigbar ist, der derart mit der Steuereinheit gekoppelt ist, dass der auf den Schlauch wirkende Klemmdruck unabhängig von der Heizeinrichtung einstellbar ist,
wobei die Steuereinheit eingerichtet ist, während eines Dosiervorgangs den Volumenstrom des Mediums durch den Verbindungsschlauch bei inaktiver Heizeinrichtung dadurch zu steuern, dass der von den Klemmbacken auf den Verbindungsschlauch ausgeübte Klemmdruck entsprechend gesteuert wird, und nach Abschluss des Dosiervorgangs die Heizeinrichtung zu aktivieren und den Verbindungsschlauch unter Aufbringung eines ihn verschließenden Klemmdrucks zu verschweißen.

Bevorzugte Ausführungsformen sind Gegenstand der abhängigen Ansprüche.

Wesentlich im Hinblick auf die erfindungsgemäße Dosiervorrichtung ist die Möglichkeit, mittels einer Heizeinrichtung, die wenigstens einer der Klemmbacken zugeordnet ist, den geklemmten Verbindungsschlauch zu erhitzen, wobei das Erhitzen selbstverständlich bis mindestens zur Erweichungstemperatur des Verbindungsschlauches möglich sein muss. Mit anderen Worten wird das bei gattungsgemäßen Dosiervorrichtungen ohnehin vorhandene Steuerventil um die zusätzliche Fähigkeit erweitert, den geklemmten Schlauch dauerhaft zu verschweißen.

Durch die vorliegende Erfindung wird es ermöglicht, den Verschlussvorgang durch Verschweißen in den Dosierprozess - gleich ob im Rahmen einer Abfüllung, einer Mischung, einer Anwendung oder Ähnliches - zu integrieren. Insbesondere ist der Einsatz eines zusätzlichen Gerätes nicht erforderlich. Auch ein zusätzlicher Arbeitsgang, insbesondere unter Einbeziehung manueller Verfahrensschritte, ist nicht notwendig. Die Vorteile im Hinblick auf Kosteneinsparung und Verbesserung der hygienischen Sicherheit sind offensichtlich.

Die spezielle Gestaltung der Heizeinrichtung, mittels deren der geklemmte Schlauch erhitzt werden kann, ist für die vorliegende Erfindung nicht relevant. Beispielsweise kann der Schlauch direkt erhitzt werden, indem zwischen den Klemmbacken ein hochfrequentes elektrisches Feld erzeugt wird, dessen Frequenz auf eine Resonanzfrequenz von Moleküldipolen des Schlauchmaterials abgestimmt ist. Alternativ kann der Schlauch indirekt geheizt werden, indem die ihn klemmenden Klemmbacken z.B. durch eine integrierte Widerstandsheizung erhitzt werden. Auch können die Klemmbacken als Reibschweiß- oder Ultraschall-Schweißköpfe ausgestaltet sein, die im geklemmten Schlauch mikroskopische Relativbewegungen und damit Hitze bewirkende Reibung erzeugen. Weiter ist die Erhitzung durch Strahlung, z.B. infraroter oder Mikrowellen-Strahlung denkbar.

Bei der Erfindung ist, wie auch bereits aus dem Stand der Technik bekannt, vorgesehen, dass der Klemmdruck, den die Klemmbacken der Schweißvorrichtung auf die Klemmstelle ausüben, variabel und insbesondere gezielt steuerbar ist. Der Fachmann wird erkennen, dass es hier zu keiner expliziten Vorgabe des Klemmdrucks bedarf. So kann der Klemmdruck auch mittelbar, etwa durch explizite Vorgabe eines Klemmbackenabstandes oder eines Akutatorvorschubs o.ä. eingestellt werden.

Weiterhin sieht die Erfindung, wie ebenfalls bereits bekannt, vor, wenigstens eine der Klemmbacken mit einem steuerbaren Aktuator zu versehen, der beispielsweise elektrisch, pneumatisch, hydraulisch oder magnetisch betreibbar ist. Wichtig ist, dass der Aktuator bidirektional steuerbar ist, d.h. sowohl zur Erhöhung des Klemmdrucks als auch zur Verringerung des Klemmdrucks ansteuerbar ist. Der Begriff des Aktuators ist hier weit zu verstehen und umfasst insbesondere auch Systeme von mehreren jeweils unidirektional arbeitenden, jedoch antagonistisch zusammenwirkenden Stellelementen.

Ferner ist vorgesehen, dass die Ansteuerung des Aktuators unabhängig von der Heizeinrichtung erfolgen kann. Durch diese ebenfalls bereits aus dem Stand der Technik bekannte Maßnahme, kann der Klemmdruck, insbesondere mittelbar über den Abstand der Klemmbacken, unabhängig von der Heizeinrichtung und insbesondere bei inaktiver Heizeinrichtung beliebig eingestellt werden. Auf diese Weise lässt sich das freie Lumen des geklemmten Schlauchs zwischen einem maximalen Öffnungszustand und einem vollständigen Verschlusszustand ansteuern, ohne dass damit zwangsläufig ein Schweißprozess verbunden wäre.

Selbstverständlich weist auch die bei der Erfindung zum Einsatz kommende Schweißvorrichtung, wie die Schweißvorrichtungen gemäß dem Stand der Technik, die Fähigkeit auf, das Schlauchmaterial bis mindestens zu dessen Erweichungstemperatur zu erhitzen. Hierbei ist ein Klemmdruck, der hoch genug ist, das Lumen des Schlauches vollständig zu verschließen, ansteuerbar, wodurch die Klemmvorrichtung unabhängig voneinander als steuerbares Klemmventil und zur dauerhaften Verschweißung des Schlauches an der Klemmstelle dienen kann.

Bei der erfindungsgemäßen Verwendung einer Schweißvorrichtung als ein steuerbares Klemmventil einer automatisierten Dosiervorrichtung, ist bevorzugt vorgesehen, dass die Steuereinheit eingerichtet ist,
- während eines Dosiervorgangs einen Volumenstrom des Mediums durch den Verbindungsschlauch bei inaktiver Heizeinrichtung dadurch zu steuern, dass der von den Klemmbacken auf den Verbindungsschlauch ausgeübte Klemmdruck entsprechend gesteuert wird, und
- nach Abschluss des Dosiervorgangs die Heizeinrichtung zu aktivieren und den Verbindungsschlauch unter Aufbringung eines ihn verschließenden Klemmdrucks zu verschweißen.

Bei einer bevorzugten Weiterbildung der erfindungsgemäßen Dosiervorrichtung ist vorgesehen, dass wenigstens eine der Klemmbacken eine mit der Steuereinrichtung gekoppelte Trennvorrichtung zum Durchtrennen des Verbindungsschlauchs aufweist. Beispielsweise kann die Trennvorrichtung eine verfahrbare Schneidklinge umfassen.

Das Vorsehen einer Trennvorrichtung erlaubt es nämlich bei der erfindungsgemäßen Verwendung einer Schweißvorrichtung als ein steuerbares Klemmventil einer automatisierten Dosiervorrichtung, dass der Verbindungsschlauch nach erfolgtem Verschweißen an der Schweißstelle durchtrennt wird. Hierzu ist die Steuereinrichtung der erfindungsgemäßen Dosiervorrichtung bevorzugt eingerichtet, die Trennvorrichtung nach erfolgter Verschweißung zur Durchtrennung des Verbindungsschlauchs anzusteuern. Mit anderen Worten ist bevorzugt vorgesehen, den geklemmten Schlauch nach seiner Verschweißung an der Schweißstelle zu durchtrennen. Der abgefüllte Beutel kann dadurch vereinzelt und zuverlässig dauerhaft verschlossen separat weiterbehandelt, beispielsweise verkauft werden. Alternativ zur Durchtrennung des Schlauches mittels einer Schneidklinge kann der Schlauch auch durch weitere Erhitzung soweit aufgeschmolzen werden, dass eine Durchtrennung erfolgt.

Weitere Merkmale und Vorteile der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibung der Zeichnungen

Es zeigen:
- Figur 1:: eine erfindungsgemäße Dosiervorrichtung,
- Figur 2:: eine Ausführungsform eines Schweiß-/Klemmventils in drei unterschiedlichen Stellungen,
- Figur 3:: eine erste Ausführungsform eines magnetisch betätigten Schweiß-/Klemmventils,
- Figur 4:: eine zweite Ausführungsform eines magnetisch betätigten Schweiß-/Klemmventils,
- Figur 5:: eine erste Ausführungsform eines pneumatisch/hydraulisch betätigten Schweiß-/Klemmventils,
- Figur 6:: eine zweite Ausführungsform eines pneumatisch/hydraulisch betätigten Schweiß-/Klemmventils,
- Figur 7:: eine Ausführungsform eines motorisch betätigten Schweiß-/Klemmventils,
- Figur 8:: eine schematische Darstellung elektroresistiv beheizter Klemm-/Schweißköpfe,
- Figur 9:: eine schematische Darstellung eines magnetisch betriebenen Klemm-/Reibschweißkopfes,
- Figur 10:: eine schematische Darstellung eines motorisch betriebenen Klemm-/Reibschweißkopfes,
- Figur 11:: eine schematische Darstellung elektrischer HF-Klemm-/Schweißköpfe,
- Figur 12:: eine schematische Darstellung einer integrierten Schlauch-Trennvorrichtung.

### Ausführliche Beschreibung bevorzugter Ausführungsformen

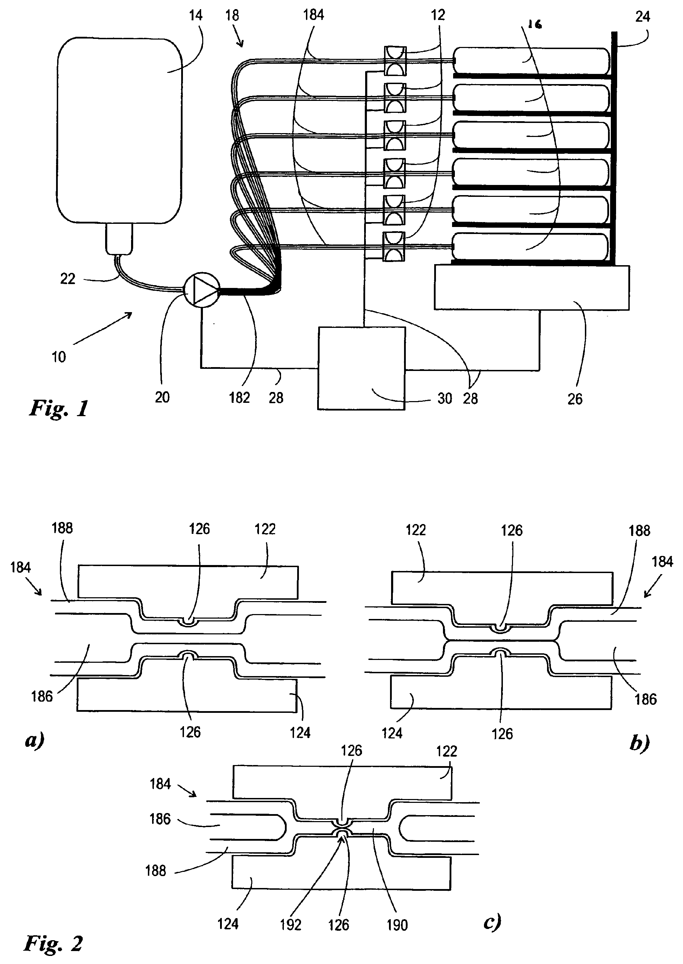

Figur 1 zeigt eine schematische Darstellung einer Dosiervorrichtung, bei der Schweißvorrichtungen als Steuerventile Einsatz finden. Derartige Vorrichtungen, die sowohl zur Steuerung eines Volumenflusses durch einen Schlauch als auch zum verschließenden Verschweißen desselben geeignet sind, sollen nachfolgend als Schweißventile 12 bezeichnet werden.

Zweck der Dosiervorrichtung 10 ist es, eine Dosierflüssigkeit aus einem großvolumigen Vorratsbehälter 14 in eine Mehrzahl von Zielbehältern 16 kleineren Volumens abzufüllen. Dabei soll eine jeweils abzufüllende Menge sehr genau eingehalten werden. Die Zielbehälter 16 sind als flexible Kunststoffbeutel ausgestaltet, die - vorzugsweise einstückig oder stoffschlüssig - mit einem Verbindungsschlauchsystem 18 verbunden sind. Das Verbindungsschlauchsystem 18 besteht bei der gezeigten Ausführungsform aus einem gemeinsamen Schlauchansatz 182, der sich in eine Vielzahl von Einzelschläuchen 184 aufspaltet. Der gemeinsame Schlauchansatz 182 ist mit dem Ausgang einer Pumpe 20 verbunden. Jeder Einzelschlauch 184 ist mit einem ihm jeweils zugeordneten Zielbeutel 16 verbunden. Vor seiner Verbindungsstelle mit dem jeweiligen Zielbeutel 16 ist jeder Verbindungsschlauch 184 zwischen den Klemmbacken eines Schweißventils 12 eingeklemmt. Die Pumpe 20, an deren Ausgang der gemeinsame Schlauchansatz 182 des Schlauchsystem 18 angeschlossen ist, ist eingangsseitig über einen weiteren Verbindungsschlauch 22 mit dem Vorratsbehälter 14 verbunden.

Die Zielbeutel 16 sind bei der gezeigten Ausführungsform auf einem Stapelregal 24 gelagert, welches seinerseits auf einer Waage 26 angeordnet ist. Die Waage 26, die Schweißventile 12 und bevorzugt, wie in der dargestellten Ausführungsform gezeigt, auch die Pumpe 20 sind über Steuerleitungen 28 mit einer Steuereinheit 30 verbunden. Die Steuereinheit 30 empfängt Wägesignale von der Waage 26 und sendet nach vorgegebenen Regeln Steuerbefehle an die Schweißventile 12 und vorzugsweise an die Pumpe 20. Die Verfahrensregeln, nach denen die Auswertung der Wägesignale und die Ansteuerung der Schweißventile 12 und der Pumpe 20 erfolgen, sind in der Steuereinheit 30 vorzugsweise als Software hinterlegt. Ein typischer Verfahrensablauf bei der gezeigten Dosiervorrichtung könnte wie folgt vor sich gehen: Im Ausgangszustand, d.h. wenn alle Schlauchverbindungen in der gezeigten und oben erläuterten Weise etabliert und die einzelnen Verbindungsschläuche 184 zwischen den Klemmbacken der Schweißventile 12 eingeklemmt sind, werden alle Schweißventile 12 auf "zu" angesteuert, d.h. ihre Klemmbacken werden soweit zusammengefahren, dass das Lumen des eingeklemmten Verbindungsschlauchs 184 vollständig verschlossen ist. Ein Volumenstrom durch das Verbindungsschlauchsystem 18 ist in diesem Zustand nicht möglich. Vor Beginn des eigentlichen Dosiervorgangs wird die Waage 26 auf einen Grundwert, vorzugsweise "Null" tariert. Alsdann wird die Pumpe 20 gestartet, wobei ein in Figur 1 nicht dargestellter Entlüftungsschlauch vorgesehen sein kann, durch den im Verbindungsschlauchsystem 18 befindliche Luft ausgeblasen werden kann. Zur Befüllung eines ersten Zielbeutels 16, beispielsweise des in Figur 1 zu unterst dargestellten Zielbeutels 16 wird das entsprechende Schweißventil 12 geöffnet, d.h. seine Klemmbacken werden soweit auseinandergefahren, dass wenigstens ein Teillumen des entsprechenden Verbindungsschlauchs 184 freigegeben und ein Volumenstrom der Dosierflüssigkeit von dem Vorratsbehälter 14 in den aktiven Zielbeutel 16 ermöglicht wird. Während des Füllvorgangs wird der Gewichtszuwachs der gesamten auf der Waage 26 positionierten Anordnung in kurzen zeitlichen Abständen, vorzugsweise quasi-kontinuierlich, gemessen und die Messergebnisse an die Steuereinheit 30 gemeldet. Diese reguliert den Volumenstrom durch entsprechende Steuerung des aktiven Schweißventils 12, wobei dem Fachmann typische Steueralgorithmen zur Realisierung eines gravimetrischen Dosierverfahrens mittels eines Steuerventils bekannt sind. Zum Abschluss des Abfüllvorgangs wird das aktive Schweißventil 12 wieder verschlossen, d.h. seine Klemmbacken soweit zusammengefahren, dass das Lumen des entsprechenden Verbindungsschlauchs 184 wieder vollständig verschlossen ist.

In einem nächsten Verfahrensschritt wird die Heizeinrichtung des aktiven Schweißventils aktiviert. Hierdurch wird der Verbindungsschlauch 184 wenigstens bis zu seiner Erweichungstemperatur erhitzt, wobei der auf ihn von den Klemmbacken des Schweißventils 12 ausgeübte Klemmdruck aufrechterhalten wird. Dabei ist es nicht zwingend erforderlich, den Klemmdruck genau konstant zu halten. Je nach Wahl des Schlauchmaterials, Wandstärke, Querschnitt, Heizverfahren etc. ist auch eine Variation des Klemmdrucks zum Beispiel in Abhängigkeit der Temperatur oder der Materialerweichung denkbar. Entscheidend ist lediglich, dass der Klemmdruck nicht soweit verringert wird, dass sich das Lumen des Schlauches wieder öffnet. Durch die Erweichung des Materials kommt es zum Verschweißen, d.h. zur stoffschlüssigen Verbindung der zusammengeklemmten Innenwände des Verbindungsschlauchs 184. Der Verbindungsschlauch 184 des gerade befüllten Zielbeutels 16 ist dann dauerhaft verschlossen. Abschließend kann, je nach spezieller Ausgestaltung des Schweißventils 12, der befüllte Zielbeutel 16 vom übrigen Schlauchsystem 184 abgetrennt werden, indem die erzeugte Schweißstelle durchtrennt wird.

Im Anschluss wird das für einen Zielbeutel 16 beschriebene Verfahren sukzessiv für die übrigen Zielbeutel 16 wiederholt, wobei jeder neue Abfüllvorgang mit einer Neutarierung der Waage 26 eingeleitet werden kann. Die Pumpe 20 kann kontinuierlich durchlaufen oder für jeden Abfüllvorgang neu gestartet und nach Abfüllung wieder gestoppt werden. Alle wesentlichen Parameter der Abfüllung werden vorzugsweise von der Steuereinheit 30 gespeichert und können zum Beispiel als Klebeetiketten für die einzelnen Beutel mittels eines angeschlossenen Druckers ausgedruckt werden.

Figur 2 zeigt in schematischer Darstellung ein Schweißventil 12, zwischen dessen Klemmbacken 122 und 124 ein Verbindungsschlauch 184 eingeklemmt ist, in drei unterschiedlichen Arbeitsphasen.
Figur 2a zeigt eine Arbeitsphase, in der die Klemmbacken 122, 124 so weit zusammengefahren sind, dass das frei Lumen 186 des Verbindungsschlauchs 184 zwischen den Klemmbacken 122, 124 zwar deutlich verengt ist, einen (reduzierten) Volumenstrom durch den Verbindungsschlauch 184 jedoch noch zulässt. In Figur 2b sind die Klemmbacken 122, 124 weiter zusammengefahren, sodass sich die Innenseiten der Schlauchwände 188 berühren. D.h. das Lumen 186 des Schlauchs ist im Bereich zwischen den Klemmbacken 122, 124 vollständig verschlossen. Ein Volumenstrom durch den Verbindungsschlauch 184 ist in diesem Zustand nicht möglich. Allerdings kann das Lumen 186 durch Öffnen, d.h. Auseinanderfahren der Klemmbacken 122, 124 erneut geöffnet werden. In Figur 2c schließlich ist eine Phase gezeigt, in der die Klemmbacken 122, 124 maximal zusammengefahren und die in Figur 2 nicht gesondert dargestellte Heizvorrichtung aktiviert ist. Durch die Aufheizung des Verbindungsschlauchs 184 bis mindestens zu seiner Erweichungstemperatur werden die einander kontaktierenden Schlauchwände 188 miteinander verschweißt. Es resultiert eine mit dem Bezugszeichen 190 versehene Schweißstelle, in der das Lumen 186 des Verbindungsschlauchs 184 durch Stoffschluss seiner Schlauchwände 188 dauerhaft verschlossen ist.

Bei der gezeigten Ausführungsform weisen die Klemmbacken 122, 124 an gegenüberliegenden Stellen jeweils einen Trennvorsprung 126 auf. Zwischen den Trennvorsprüngen 126 wird der Verbindungsschlauch 124 stärker komprimiert, als im Bereich der übrigen Schweißstelle 190. Hierdurch fließt das erweichte Schlauchmaterial zwischen den Trennvorsprüngen 126 nach außen, sodass es zu einer vollständigen Durchtrennung an der Trennstelle 192 kommt. Dieser Vorgang kann dadurch unterstützt werden, dass die Heizvorrichtung so gestaltet ist, dass das Schlauchmaterial zwischen den Verbindungsvorsprüngen 126 stärker erwärmt wird als zwischen den übrigen Bereichen der Klemmbacken 122, 124.

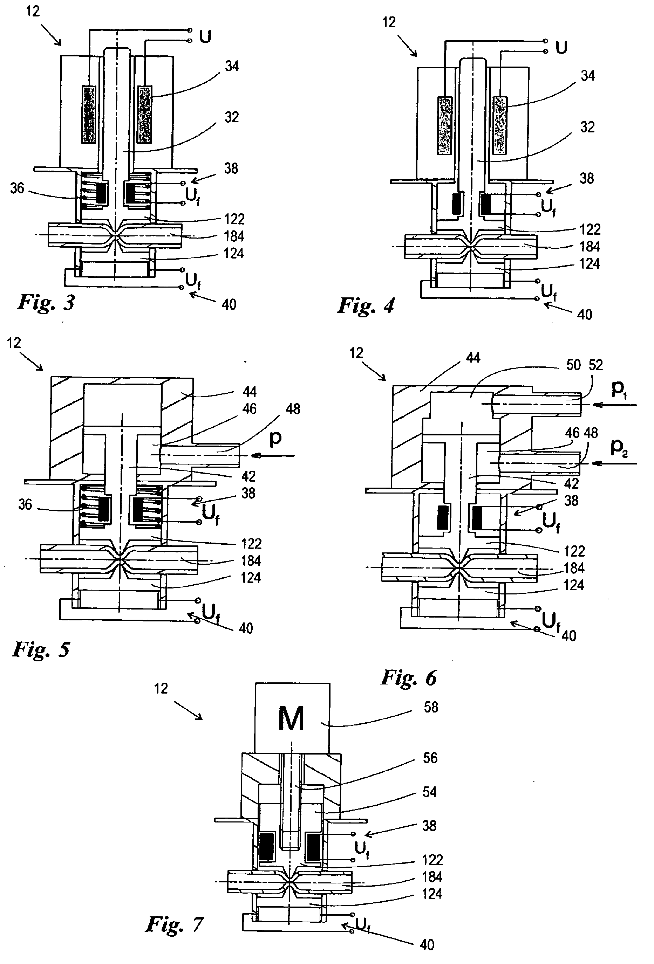

Figur 3 zeigt in größerem Detail eine erste Ausführungsform eines Schweißventils. Bei dieser Ausführungsform ist seine Funktion als Steuerventil durch einen federvorgespannten elektromagnetischen Antrieb realisiert. An die in Figur 3 obere Klemmbacke 122 schließt sich ein vorzugsweise als Weicheisenkern ausgebildeter Stößel 32 an, der von einer Spule 34 umgeben ist. Die Spule 34 ist an eine steuerbare Gleichspannungsquelle angeschlossen, mit der der Hub des Stößels 32 und damit der Klemmdruck, den die Klemmbacke 122 in Zusammenwirkung mit der in Figur 3 unteren Klemmbacke 124 auf den eingeklemmten Schlauch 184 ausübt, gesteuert werden kann. Bei der gezeigten Ausführungsform ist die Klemmbacke 122 mittels einer Feder 136 derart federvorgespannt, dass das Ventil bei stromloser Spule 34 auf "zu" geschaltet ist, d.h. der Elektromagnet 32/34 wirkt antagonistisch zur Feder 136.

### Weiter sind in Figur 3 zwei unterschiedliche

### Heizeinrichtungen dargestellt.

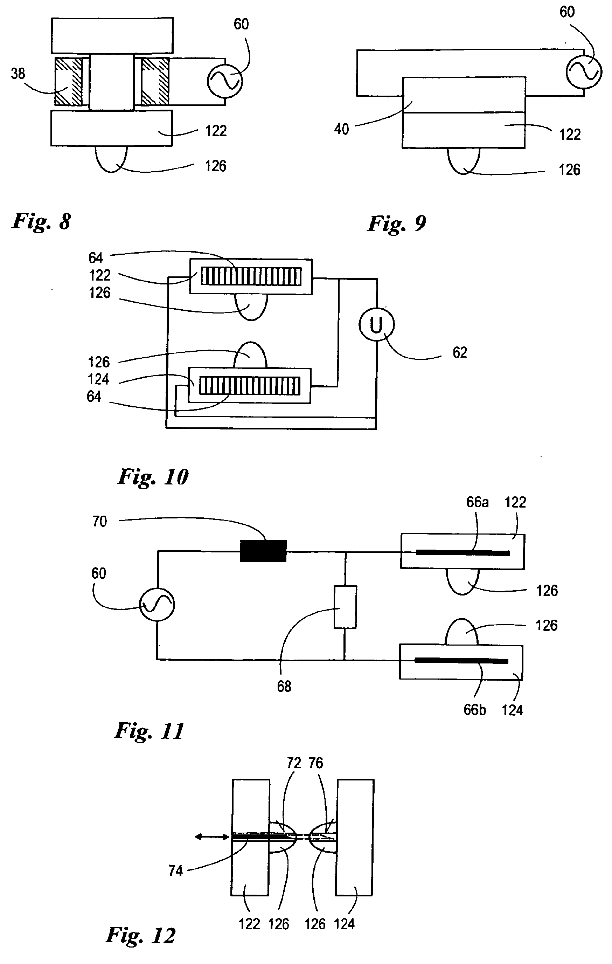

Der in Figur 3 oberen Klemmbacke 122 ist eine elektromagnetische Vibrationsheizung 38 und der in Figur 3 unteren Klemmbacke 124 ist eine piezoelektrische Vibrationsheizung 40 zugeordnet. Details dieser Varianten sollen weiter unten im Zusammenhang mit den Figuren 8 und 9 beschrieben werden. Man beachte, dass die dargestellten Heizeinrichtungen 38, 40 vorzugsweise nicht wie in Figur 3 suggeriert gemeinsam in einem Schweißventil 12 realisiert sind. Vielmehr wird bevorzugt lediglich eine Art der Heizeinrichtung in einem Schweißventil 12 verwirklicht. Dabei ist es sowohl möglich, beide Klemmbacken 122, 124 mit je einer Heizeinrichtung auszustatten, als auch lediglich eine der Klemmbacken 122, 124 mit einer Heizeinrichtung zu versehen. Derartige Fragen der speziellen Auslegung wird der Fachmann jedoch jeweils in Ansehung des konkreten Problems ohne weiteres lösen können.

Figur 4 zeigt eine Modifikation des Schweißventils 12 von Figur 3. Bei dieser Ausführungsform ist keine Vorspannfeder 36 vorgesehen. Dieses Schweißventil 12 wird daher im stromlosen Zustand aufgrund der Eigenelastizität des eingeklemmten Schlauchs selbstständig in den Zustand "offen" übergehen. Im Übrigen gilt das oben zu dem Schweißventil 12 der Figur 3 gesagte.

Figur 5 zeigt eine alternative Realisierung der Steuerventilfunktion des Schweißventils. Die in Figur 5 obere Klemmbacke 122 geht in einen Kolben 42 über, der in einem Gehäuse 44 eine Druckkammer 46 bildet. Die Druckkammer 46 ist mit einer Druckleitung verbunden, durch die ein Druckmedium, beispielsweise eine Druckflüssigkeit zur Realisierung eines hydraulischen oder ein Druckgas zur Realisierung eines pneumatischen Systems in die Druckkammer 46 geleitet werden kann. Bei Erhöhung des Drucks in der Druckkammer 46 wird der Kolben 42 gegen den Druck der die Klemmbacke 122 in Richtung "zu" vorspannenden Feder 36 nach oben angehoben, d.h. der Klemmdruck auf den eingeklemmten Schlauch wird verringert. Reduktion des Drucks in der Druckkammer 46 verringert die auf die Feder 36 wirkende Hubkraft, sodass sich die Klemmbacke 122 senkt. Im drucklosen Zustand ist das Schweißventil 12 somit in seiner "zu"-Stellung. Da es bei dieser Ausführungsform keine Möglichkeit gibt, den Klemmdruck hydraulisch oder pneumatisch über den Grunddruck der Feder 36 zu erhöhen, ist die Feder 36 vorzugsweise so stark auszulegen, dass sie allein ausreicht, um einen vollständigen Lumenverschluss des angeklemmten Schlauchs sowie einen für den Schweißvorgang erforderlichen Klemmdruck zu gewährleisten. Im Übrigen gilt das oben zu dem Schweißventil 12 der Figur 3 gesagte.

Figur 6 zeigt eine Modifikation des Schweißventils 12 von Figur 5. Bei dieser Ausführungsform ist keine Vorspannfeder 36 vorgesehen, sodass der Kolben 42 sowohl in "zu"- als auch in "offen"-Richtung pneumatisch bzw. hydraulisch zu betätigen ist. Hierzu ist ein weiterer Druckraum 50 oberhalb des Kolbens 42 vorgesehen, der mit einer zweiten Druckleitung 52 verbunden ist. Der auf den eingeklemmten Schlauch wirkende Klemmdruck wird somit im Wesentlichen durch den Differenzdruck in den Druckkammern 46 und 50 gesteuert. Im Übrigen gilt das oben zu dem Schweißventil von Figur 5 gesagte.

Figur 7 zeigt eine Ausführungsform eines Klemmventils 12, bei der die Steuerventilfunktion mittels eines mechanischen Spindeltriebs realisiert ist. Hierzu ist die in Figur 7 obere Klemmbacke 122 mit einer Gewindemutter 54 verbunden, in deren Innengewinde eine Gewindespindel 56 angeordnet ist, die ihrerseits mittels eines Motors 58 um ihre Längsachse rotierbar ist. Eine Vorspannung z.B. mittels einer Vorspannfeder ist bei dieser Ausführungsform nicht vorgesehen. Bei einer entsprechend steilen Gestaltung der Gewinde der Gewindemutter 54 und der Gewindespindel 56 kann jedoch auch eine mechanische Vorspannung sinnvoll sein. Im Übrigen gilt das oben zu dem Schweißventil 12 der Figur 3 gesagte.

Figur 8 zeigt als grob schematische Darstellung eine elektromagnetische Vibrationsheizung, die einer Klemmbacke 122 eines Schweißventils 12 zugeordnet sein kann. Bei der gezeigten Ausführungsform weist die Klemmbacke 122 einen von einer Spule 38 umgebenden Weicheisen- oder Permanentsockel auf. Die Spule 38 ist mit einer Wechselstromquelle 60 verbunden, sodass bei Bestromung der Spule 38 eine axiale Vibration der Klemmbacke 122 erfolgt. Diese Vibration, die von dem Material eines eingeklemmten Schlauches weggepuffert wird, führt in diesem zu Reibung, sodass er sich erwärmt. Bei hinreichender Erhitzung erfolgt eine Materialerweichung, die bei hinreichend hohem Grunddruck zu einer Verschweißung des eingeklemmten Schlauchs 184 führt. Alternativ könnte die Klemmbacke 122 auch um ihre Längsachse rotierbar gelagert sein und ihr Sockel nach Art eines Elektromotors in Umfangsrichtung beabstandete Magnetsegmente aufweisen, die von einer entsprechenden Spulenanordnung umgeben sind. Durch entsprechende Ansteuerung der Bestromung kann eine Rotation oder rotative Vibration der Klemmbacke 122 erzeugt und der eingeklemmte Schlauch durch die resultierende Reibung erhitzt werden.

Figur 9 zeigt eine alternative Variante zur Heizung eines eingeklemmten Schlauchs durch Reibung. Bei dieser Ausführungsform ist die Klemmbacke 122 auf einem piezoelektrischen Sockel montiert, der von einer geeigneten Wechselspannung beaufschlagt wird. Durch den piezoelektrischen Effekt dehnt sich und schrumpft der Piezo-Sockel, sodass eine Vibration der Klemmbacke 122 resultiert. Je nach spezieller Ausgestaltung des Piezo-Sockels 40 können unterschiedliche Vibrationen der Klemmbacke 122, z.B. axial, lateral oder rotativ realisiert werden.

Figur 10 zeigt eine alternative Heizvorrichtung, bei der die Klemmbacken 122, 124 mit Widerstandsheizelementen 64 ausgestattet sind. Diese sind mit einer Gleich- oder Wechselspannungsquelle verbunden und heizen sich in bekannter Weise auf. Bei geeigneter Materialwahl der Klemmbacken 122, 124 mit vorzugsweiser hoher Wärmeleitfähigkeit, beispielsweise Kupfer oder Messing, kann die elektrisch erzeugte Wärme leicht und gezielt auf den eingeklemmten Schlauch übertragen werden.

Figur 11 zeigt eine elektrische Hochfrequenzheizung, bei der die Klemmbacken 122, 124 als "Platten" 66a, 66b eines elektrischen Plattenkondensators wirken. Der Kondensator ist Bestandteil eines elektrischen Schwingkreises, der weitere elektrische Elemente, wie beispielsweise einen ohmschen Widerstand 68 und eine Spule 70 umfassen kann. Der elektrische Schwingkreis wird von einer Wechselspannungsquelle vorzugsweise in Resonanz angeregt. Ist die Resonanzfrequenz des Schwingkreises auf die Resonanzfrequenz von Moleküldipolen im Wandmaterial des Schlauchs abgestimmt, können diese durch das über dem Kondensator anliegende Wechselfeld zu Schwingungen angeregt werden, was sich in einer Aufheizung des Wandmaterials äußert.

Figur 12 zeigt eine bevorzugte Weiterbildung der Erfindung, die unabhängig von der Realisierung der Steuerventilfunktion und der Realisierung der Heizfunktion ist. In der in Figur 20 linken Klemmbacke 122, ist ein axialer Schneidenkanal 74 angeordnet. Der Schneidenkanal 74 birgt eine axial bewegliche Schneidklinge 72. Diese ist mittels eines nicht dargestellten Antriebs axial verschiebbar und zwar zwischen einer Schutzstellung, die in Figur 12 durch eine schwarz ausgefüllte Klinge 72 dargestellt ist, und einer Schneidstellung, die in Figur 12 als gestrichelt umrandete Klinge 72 dargestellt ist. In der Schutzstellung ist die Schneidkante der Schneidklinge 72 in die Klemmbacke 122 zurückgezogen. In der Schneidstellung ist sie über die Vorderkante der Klemmbacke 122 hinausgeschoben und wird vorzugsweise in einem korrespondierenden Aufnahmekanal 76 der gegenüberliegenden Klemmbacke 124 aufgenommen. Der Fachmann erkennt, dass ein in Figur 12 nicht gezeigter, zwischen den Klemmbacken 122, 124 eingeklemmter Schlauch in dieser Schneidstellung durchtrennt ist. Die Durchschneidung erfolgt vorzugsweise nach Abschluss der Verschweißung des eingeklemmten Schlauchs im Zentrum der resultierenden Schweißstelle.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum an Variationsmöglichkeiten an die Hand gegeben. Insbesondere kann die Form der Klemmbacken 122, 124 auf die gewünschte Form der Schweißstelle abgestimmt sein und von den hier dargestellten Formen deutlich abweichen. Auch die konkrete Wahl der Realisierung der Steuerventil-Funktion ist ebenso für die Erfindung nicht von Belang wie die spezielle Realisierung der Heiz- bzw. Schweißfunktion. Auch kann die erfindungsgemäße Dosierfunktion anders als dargestellt, insbesondere mit mehr oder weniger Zielbehältern 16 und/oder mehr Vorratsbehältern 14 sowie mit zusätzlichen oder alternativen, nicht dargestellten Komponenten ausgelegt sein. Beispielsweise kann der Volumenstrom von dem oder den Vorratsbehältern 14 zu den Zielbeuteln 16 auch ohne Verwendung einer Pumpe 20, etwa durch Druckbeaufschlagung des oder der Zielbehälter 16 oder schwerkraftgetrieben erfolgen. Anstelle eines Stapelregals 24, in dem die Zielbeutel 16 übereinander angeordnet sind, kann auch eine Anordnung nebeneinander, z.B. in einem Hängegestell zum Einsatz kommen.

### Bezugszeichenliste

- 10: Dosiervorrichtung
- 12: Schweißventil
- 122: erste Klemmbacke von 12
- 124: zweite Klemmbacke von 12
- 126: Trennvorsprung von 122/124
- 14: Vorratsbehälter/Vorratsbeutel
- 16: Zielbehälter/Zielbeutel
- 18: Verbindungsschlauchsystem
- 182: gemeinsamer Schlauchansatz
- 184: Einzelverbindungsschlauch
- 186: Lumen von 184
- 188: Schlauchwand von 184
- 190: Schweißstelle/Klemmstelle
- 192: Trennstelle
- 20: Pumpe
- 22: Schlauch
- 24: Regalgestell
- 26: Waage
- 28: Steuerleitung
- 30: Steuereinheit
- 32: Weicheisenschaft
- 34: Magnetspule
- 36: Vorspannfeder
- 38: elektromagnetische Reibheizung/Magnet-Spule
- 40: piezoelektrische Reibheizung/Piezo-Sockel
- 42: Kolben
- 46: Druckraum
- 48: Druckleitung
- 50: Druckraum
- 52: Druckleitung
- 54: Gewindemutter
- 56: Gewindespindel
- 58: Motor
- 60: Wechselspannungsquelle
- 62: Spannungsquelle
- 64: Widerstandsheizelement
- 66a,b: Kondensatorplatten
- 68: Widerstandselement
- 70: Spule
- 72: Schneidkanal
- 74: Schneidklinge
- 76: Aufnahmekanal

## Patentansprüche

1. Verwendung einer Schweißvorrichtung zum verschließenden Verschweißen von thermoplastischen Kunststoffschläuchen (184), umfassend eine Klemmvorrichtung (12) mit wenigstens zwei Klemmbacken (122, 124), von denen wenigstens eine bewegbar ist und zwischen denen ein zu verschweißender Schlauch (184) einklemmbar ist,
wobei die Klemmvorrichtung (12) eine mit einer Steuereinheit (30) gekoppelte Heizeinrichtung (38; 40; 64) zum Erhitzen des zwischen den Klemmbacken (122, 124) eingeklemmten Schlauches aufweist, und
die wenigstens eine bewegbare Klemmbacke (122) mittels eines steuerbaren, bidirektionalen Aktuators (32, 34; 42-52; 54, 56, 58) betätigbar ist, der derart mit der Steuereinheit (30) gekoppelt ist, dass der auf den Schlauch (184) wirkende Klemmdruck unabhängig von der Heizeinrichtung einstellbar ist,
als ein steuerbares Klemmventil einer automatisierten Dosiervorrichtung (10), mittels derer ein Medium von einem Vorratsbehälter (14) durch einen zwischen den Klemmbacken eingeklemmten, aus thermoplastischem Kunststoff bestehenden Verbindungsschlauch (184) dosiert in einen Zielbehälter (16) überführbar ist.

2. Verwendung nach Anspruch 1,
**dadurch gekennzeichnet,**
**dass** die Steuereinheit (30) eingerichtet ist, während eines Dosiervorgangs einen Volumenstrom des Mediums durch den Verbindungsschlauch (184) bei inaktiver Heizeinrichtung (38; 40; 64) dadurch zu steuern, dass der von den Klemmbacken (122, 124) auf den Verbindungsschlauch (184) ausgeübte Klemmdruck entsprechend gesteuert wird, und nach Abschluss des Dosiervorgangs die Heizeinrichtung (38; 40; 64) zu aktivieren und den Verbindungsschlauch (184) unter Aufbringung eines ihn verschließenden Klemmdrucks zu verschweißen.

3. Verwendung nach Anspruch 2,
**dadurch gekennzeichnet,**
**dass** der Verbindungsschlauch (184) nach erfolgtem Verschweißen an der Schweißstelle (190) durchtrennt wird.

4. Automatisierte Dosiervorrichtung zum dosierten Überführen eines Mediums von einem Vorratsbehälter (14) durch einen aus thermoplastischem Kunststoff bestehenden Verbindungsschlauch (18, 184) in einen Zielbehälter (16), wobei der Verbindungsschlauch (184) zwischen wenigstens zwei Klemmbacken (122, 124), von denen wenigstens eine bewegbar ist, einer mit einer Steuereinheit (30) gekoppelten, steuerbaren Klemmvorrichtung einer Schweißvorrichtung (12), positionierbar ist, sodass ein Volumenstrom des Mediums durch Steuerung eines von der Klemmvorrichtung (12) an einer Klemmstelle (190) auf den Verbindungsschlauch (184) ausgeübten Klemmdrucks steuerbar ist,
wobei wenigstens eine der Klemmbacken (122, 124) eine mit der Steuereinheit (30) gekoppelte Heizeinrichtung (38; 40; 64) zum Erhitzen des Verbindungsschlauches (184) aufweist und die wenigstens eine bewegbare Klemmbacke (122) mittels eines steuerbaren, bidirektionalen Aktuators (32, 34; 42-52; 54, 56, 58) betätigbar ist, der derart mit der Steuereinheit (30) gekoppelt ist, dass der auf den Schlauch (184) wirkende Klemmdruck unabhängig von der Heizeinrichtung (38; 40; 64) einstellbar ist, wobei die Steuereinheit (30) eingerichtet ist, während eines Dosiervorgangs den Volumenstrom des Mediums durch den Verbindungsschlauch (184) bei inaktiver Heizeinrichtung dadurch zu steuern, dass der von den Klemmbacken (122, 124) auf den Verbindungsschlauch (184)ausgeübte Klemmdruck entsprechend gesteuert wird, und nach Abschluss des Dosiervorgangs die Heizeinrichtung (38; 40; 64) zu aktivieren und den Verbindungsschlauch (184) unter Aufbringung eines ihn verschließenden Klemmdrucks zu verschweißen.

5. Dosiervorrichtung nach Anspruch 4,
**dadurch gekennzeichnet,**
**dass** wenigstens eine der Klemmbacken (122, 124) eine mit der Steuereinheit (30) gekoppelte Trennvorrichtung (72, 74, 76) zum Durchtrennen des Verbindungsschlauchs (184) aufweist.

6. Dosiervorrichtung nach Anspruch 5,
**dadurch gekennzeichnet,**
**dass** die Trennvorrichtung (72, 74, 76) eine verfahrbare Schneidklinge (74) umfasst.

7. Dosiervorrichtung nach Anspruch 5 bis 6,
**dadurch gekennzeichnet,**
**dass** die Steuereinheit (30) weiter eingerichtet ist, die Trennvorrichtung (72, 74, 76) nach erfolgter Verschweißung zur Durchtrennung des Verbindungsschlauches (184) anzusteuern.

## Claims

1. Use of a welding device for seamed welding of thermoplastic plastics material hoses (184), comprising a clamping device (12) with at least two clamping jaws (122, 124), of which at least one is movable and between which a hose (184) to be welded can be clamped in place,
wherein the clamping device (12) comprises a heating device (38; 40; 64), which is coupled with a control unit (30), for heating the hose clamped in place between the clamping jaws (122, 124), and
the at least one movable clamping jaw (122) is actuable by means of a controllable bidirectional actuator (32, 34; 42-52; 54, 56, 58), which is so coupled with the control unit (30) that the clamping pressure acting on the hose (184) is settable in dependently of the heating device, as a controllable clamping valve of an automatic metering device (10), by means of which a medium from a storage container (14) is transferrable in metered form to a target container (16) through a connecting hose (184) consisting of thermoplastic plastics material and clamped in place between the clamping jaws.

2. Use according to claim 1, **characterised in that** the control unit (30) is arranged to so control a volume flow of the medium through the connecting hose (184) during a metering process when the heating device (38; 40; 64) is inactive that the clamping pressure exerted by the clamping jaws (122, 124) on the connecting hose (184) is correspondingly controlled, after termination of the metering process, to activate the heating device (38; 40; 64) and to weld the connecting hose (184) under application of a clamping pressure closing it.

3. Use according to claim 2, **characterised in that** the connecting hose (184) is severed at the welding point (190) after welding has been carried out.

4. Automatic metering device for metered transfer of a medium from a storage container (14) through a connecting hose (18, 184), which consists of thermoplastic plastics material, to a target container (16),
wherein the connecting hose (184) is positionable between at least two clamping jaws (122, 124), of which at least one is movable, of a controllable clamping device, which is coupled with a control unit (30), of a welding device (12) so that a volume flow of the medium is controllable through controlling a clamping pressure exerted by the clamping device (12) on the connecting hose (184) at a clamping point (190),
wherein at least one of the clamping jaws (122, 124) comprises a heating device (38; 40; 64), which is coupled with the control unit (30), for heating the connecting hose (184) and the at least one movable clamping jaw (122) is actuable by means of a controllable bidirectional actuator (32, 34; 42-52; 54, 56, 58), which is so coupled with the control unit (30) that the clamping pressure acting on the hose (184) is settable in dependently of the heating device (38; 40; 64) and
wherein the control unit (30) is arranged to so control the volume flow of the medium through the connecting hose (184) during a metering process when the heating device is inactive that the clamping pressure exerted by the clamping jaws (122, 124) on the connecting hose (184) is correspondingly controlled, after termination of the metering process, to activate the heating device (38; 40; 64) and to weld the connecting hose (184) under application of a clamping force closing it.

5. Metering device according to claim 4, **characterised in that** at least one of the clamping jaws (122, 124) comprises a separating device (72, 74, 76), which is coupled with the control unit (30), for severing the connecting hose (184).

6. Metering device according to claim 5, **characterised in that** the separating device (72, 74, 76) comprises a movable knife blade (74).

7. Metering device according to claim 5 or 6, **characterised in that** the control unit (30) is further arranged to activate the separating device (72, 74, 76) for severing the connecting hose (184) after welding has been carried out.

## Revendications

1. Utilisation d'un dispositif de soudage pour le soudage de fermeture de tuyaux thermoplastiques (184), comprenant un dispositif de serrage (12) avec au moins deux mâchoires de serrage (122, 124), dont au moins une est mobile et entre lesquelles un tuyau à souder (184) peut être serré,
le dispositif de serrage (12) présentant un dispositif de chauffage (38 ; 40 ; 64) couplé à une unité de commande (30) pour le chauffage du tuyau serré entre les mâchoires de serrage (122, 124) et
l'au moins une mâchoire de serrage mobile (122) étant actionnable à l'aide d'un actionneur (32, 34 ; 42-52 ; 54, 56, 58) bidirectionnel commandable qui est couplé à l'unité de commande (30) de telle manière que la pression de serrage agissant sur le tuyau (184) puisse être réglée indépendamment du dispositif de chauffage,
en tant que soupape de serrage commandable d'un dispositif de dosage automatisé (10), à l'aide duquel un agent peut être transféré en étant dosé dans un récipient cible (16) d'un réservoir de stockage (14) par un tuyau de liaison (184) se composant d'un thermoplastique, serré entre les mâchoires de serrage.

2. Utilisation selon la revendication 1,
**caractérisée en ce**
**que** l'unité de commande (30) est aménagée afin de commander pendant un processus de dosage un débit volumétrique de l'agent par le tuyau de liaison (184) en cas de dispositif de chauffage (38 ; 40 ; 64) inactif du fait que la pression de serrage exercée par les mâchoires de serrage (122, 124) sur le tuyau de liaison (184) est commandée en conséquence et d'activer le dispositif de chauffage (38 ; 40 ; 64) à la fin du processus de dosage et de souder le tuyau de liaison (184) par l'application d'une pression de serrage le fermant.

3. Utilisation selon la revendication 2,
**caractérisée en ce**
**que** le tuyau de liaison (184) est séparé une fois le soudage effectué sur le point de soudure (190).

4. Dispositif de dosage automatisé pour le transfert dosé d'un agent d'un réservoir de stockage (14) par un tuyau de liaison (18, 184) se composant de thermoplastique dans un réservoir cible (16), le tuyau de liaison (184) étant positionnable entre au moins deux mâchoires de serrage (122, 124), dont au moins une est mobile, un dispositif de serrage commandable, couplé à une unité de commande (30) d'un dispositif de soudage (12) de sorte qu'un débit volumétrique de l'agent puisse être commandé par commande d'une pression de serrage exercée par le dispositif de serrage (12) sur un point de serrage (190) sur le tuyau de liaison (184),
au moins l'une des mâchoires de serrage (122, 124) présentant un dispositif de chauffage (38 ; 40 ; 64) couplé à l'unité de commande (30) pour le chauffage du tuyau de liaison (184) et l'au moins une mâchoire de serrage mobile (122) étant actionnable à l'aide d'un actionneur (32, 34 ; 42-52 ; 54, 56, 58) bidirectionnel commandable qui est couplé à l'unité de commande (30) de telle manière que la pression de serrage agissant sur le tuyau (184) puisse être réglée indépendamment du dispositif de chauffage (38 ; 40 ; 64), l'unité de commande (30) étant aménagée afin de commander pendant un processus de dosage le débit volumétrique de l'agent par le tuyau de liaison (184) en cas de dispositif de chauffage inactif du fait que la pression de serrage exercée par les mâchoires de serrage (122, 124) sur le tuyau de liaison (184) soit commandée en conséquence et d'activer à la fin du processus de dosage le dispositif de chauffage (38 ; 40 ; 64) et de souder le tuyau de liaison (184) par l'application d'une pression de serrage le fermant.

5. Dispositif de dosage selon la revendication 4,
**caractérisé en ce**
**que** l'au moins une des mâchoires de serrage (122, 124) présente un dispositif de séparation (72, 74, 76) couplé à l'unité de commande (30) pour la séparation du tuyau de liaison (184).

6. Dispositif de dosage selon la revendication 5,
**caractérisé en ce**
**que** le dispositif de séparation (72, 74, 76) comporte une lame de coupe déplaçable (74).

7. Dispositif de dosage selon la revendication 5 à 6,
**caractérisé en ce**
**que** l'unité de commande (30) est encore aménagée afin de commander le dispositif de séparation (72, 74, 76) une fois le soudage effectué pour la séparation du tuyau de liaison (184).
